# EUROPÄISCHE PATENTSCHRIFT

(11) **EP 4 469 116 B1**
(45) Veröffentlichungstag und Bekanntmachung des Hinweises auf die Patenterteilung: **25.02.2026**
(21) Anmeldenummer: 23702103.5
(22) Anmeldetag: 25.01.2023
(51) Int. Cl.: A61M 15/00, A61M 11/00, A61M 11/02, A61M 15/08, A61M 16/00

(54) **GERÄT ZUR VERABREICHUNG EINES AEROSOLS AN DEN NASENRAUM**
APPARATUS FOR ADMINISTERING AN AEROSOL TO THE NASAL CAVITY
APPAREIL D'ADMINISTRATION D'UN AÉROSOL À LA CAVITÉ NASALE

(30) Priorität: 26.01.2022 DE 102022000297
(43) Veröffentlichungstag der Anmeldung: 04.12.2024
(73) Patentinhaber: Smart Medical Munich UG, 81677 München (DE)
(72) Erfinder: MATL, Florian, 82131 Gauting (DE); POHL, Berthold, 82152 Krailling (DE)
(74) Vertreter: Zimmermann & Partner Patentanwälte mbB
(86) Internationale Anmeldenummer: PCT/EP2023/051818
(87) Internationale Veröffentlichungsnummer: WO 2023/144212

(56) Entgegenhaltungen:
- US-A1- 2018 036 498
- US-B2- 7 854 227
- US-B2- 8 899 230
- US-B2- 8 910 629

## Beschreibung

Die vorliegende Anmeldung betrifft ein Gerät zur Verabreichung eines Aerosols an den Nasenraum einer Person, sowie eine Aktivierungsvorrichtung und eine Aerosol-Erzeugungsvorrichtung eines solchen Geräts. Ferner beschrieben ist ein Verfahren zum Erzeugen eines Aerosols zur Verabreichung an den Nasenraum.

Ein Aerosol ist ein Gemisch aus festen und/oder flüssigen Schwebeteilchen in einem Gas. Das an den Nasenraum zu verabreichende Aerosol kann eine Substanz enthalten wie etwa ein pulverförmiges und/oder flüssiges Medikament, ein örtlich anzuwendendes Arzneimittel, ein Reinigungsmittel, eine Spüllösung und/oder Kombinationen daraus. Die Substanz kann mit dem Gerät in das Aerosol überführt und dann von dem Gerät in die Nase einer Person abgegeben und dadurch dem Nasenraum der Person zugeführt werden.

Geräte zur Verabreichung von Substanzen an den Nasenraum sind bereits bekannt. Die Druckschrift EP 2 340 865 B1 beschreibt verschiedene nasale Verabreichungsgeräte mit einer Exhalationseinheit mit Mundstück und einer Zuführungseinheit mit Nasenstück. Die in das Mundstück ausgeatmete Luft kann bspw. selbst ein Medikament mitreißen oder kann einen anderen Luftstrom generieren, der ein Medikament mitführt, und dieser das Medikament führende Luftstrom kann über ein Nasenstück in die Nase der Person abgegeben werden. Das in der genannten Druckschrift beschriebene Verabreichungsgerät ist jedoch aus medizinischer Sicht nicht immer zuverlässig, bspw. weil der Verwender durch ungewollte Variation seiner Exhalation in das Mundstück potentiell die nasale Verabreichung in unerwünschter Weise beeinflussen kann. Außerdem ist das Verabreichungsgerät umständlich in der Handhabung, und es ist schwierig, das Verabreichungsgerät flexibel an verschiedene Dosierungen, Anwendungswünsche und Aerosol-Erzeugungsmechanismen anzupassen.

Weitere Geräte mit Einblasöffnung zur Verabreichung von Substanzen in den Nasenraum sind in US 2018/036498 A1, US 7 854 227 B2, US 8 910 629 B2 und US 8 899 230 B2 offenbart.

In Anbetracht der beschriebenen Probleme ist es die Aufgabe der vorliegenden Erfindung, ein einfach handhabbares, flexibel einsetzbares und aus medizinischer Sicht zuverlässiges Gerät zur Verabreichung eines Aerosols an den Nasenraum einer Person bereitzustellen. Ferner werden eine Aktivierungsvorrichtung und eine Aerosol-Erzeugungsvorrichtung eines solchen Geräts bereitgestellt.

Diese Aufgabe wird durch ein Gerät zur Verabreichung eines Aerosols an den Nasenraum gemäß Anspruch 1 gelöst. Vorteilhafte Weiterbildungen sind in den abhängigen Ansprüchen beschrieben.

Das offenbarungsgemäße Gerät umfasst eine Aerosol-Erzeugungsvorrichtung mit einem Zerstäuber zum Erzeugen des Aerosols und eine Aktivierungsvorrichtung. Die Aktivierungsvorrichtung umfasst eine Einblasöffnung, eine erste Steuereinheit, die eingerichtet ist zum Aktivieren der Aerosol-Erzeugungsvorrichtung mittels eines Aktivierungssignals als Reaktion auf ein Ausatmen einer Person in die Einblasöffnung, und einen Blaswiderstand, der dazu eingerichtet ist, dem Ausatmen der Person einen Widerstand entgegenzusetzen.

In einer bevorzugten Ausführungsform ist die erste Steuereinheit zum drahtlosen Aktivieren der Aerosol-Erzeugungsvorrichtung, insbesondere über Funk und/oder mittels eines IR-Signals eingerichtet. Beispielsweise umfasst die erste Steuereinheit einen elektronischen Schaltkreis wie etwa einen Microcontroller, der beim Ausatmen einer Person in die Einblasöffnung das Senden des Aktivierungssignals an die Aerosol-Erzeugungsvorrichtung veranlasst, beispielsweise mit einer Sendeeinheit, insbesondere mittels einer Funk-Sendeeinheit oder eines IR-Emitters. Das Aktivierungssignal kann eine Aerosol-Erzeugung durch den Zerstäuber auslösen, wenn es von der Aerosol-Erzeugungseinheit empfangen wird. In einigen Ausführungsformen kann die erste Steuereinheit einen aktiven oder passiven Transponder aufweisen und das Aktivierungssignal ist ein Transpondersignal. In einigen Ausführungsformen kann die erste Steuereinheit einen Funk-Transmitter aufweisen, und das Aktivierungssignal ist in Form einer elektromagnetischen Welle als Funksignal übermittelbar. Das Signal kann ein auf eine Trägerwelle aufmoduliertes Nutzsignal sein, das über eine Antenne versendet wird. In einigen Ausführungsformen kann das Aktivierungssignal ein elektrisches Signal sein, das bspw. über ein Kabel - also nicht notwendigerweise drahtlos - von der Aktivierungsvorrichtung an die Aerosol-Erzeugungsvorrichtung übermittelbar ist. Optional kann eine Kommunikation zwischen der Aktivierungseinrichtung und der Aerosol-Erzeugungseinrichtung über einen bekannten Standard wie etwa Wi-Fi (WLAN) oder Bluetooth direkt oder mittelbar, bspw. über einen Router, erfolgen.

In einigen Ausführungsformen, die mit anderen hierin beschriebenen Ausführungsformen kombinierbar sind, sind die Aktivierungseinrichtung und/oder die Aerosol-Erzeugungseinrichtung an ein gemeinsamen Netzwerk, wie etwa ein WLAN oder LAN Netzwerk, angeschlossen und eine Kommunikation erfolgt über das Netzwerk. Auch eine Kommunikation über mehrere Netze, an die die Aktivierungseinrichtung bzw. die Aerosol-Erzeugungseinrichtung direkt oder mittelbar angeschlossen sein können, ist vorstellbar. Insbesondere wird das Aktivierungssignal nicht notwendigerweise von der Aktivierungseinrichtung direkt an die Aerosol-Erzeugungseinrichtung übermittelt, sondern optional indirekt oder mittelbar, bspw. über einen Switch, einen Router oder ein Gateway. Das Aktivierungssignal kann in diesem Fall das "Nutzsignal" zur Aktivierung der Aerosol-Erzeugungseinrichtung sein, das im Hinblick auf eine drahtlose Übertragung mittels eines oder mehrerer Trägersignale auf einem beliebigen Übertragungsweg über eine oder mehrere Zwischenstationen transportiert werden kann, bis es von der Aerosol-Erzeugungseinrichtung empfangen wird. Die erste Steuereinheit kann alle erforderlichen Mittel zur Kommunikation in einem oder in mehreren Netzwerken oder zur Direktkommunikation, bspw. mittels Bluetooth, aufweisen.

Das Ausatmen einer Person in die Einblasöffnung kann zweckmäßigerweise mittels eines in der Aktivierungsvorrichtung vorgesehen Sensors wie etwa eines Drucksensors oder Strömungssensors erkannt werden, woraufhin die erste Steuereinheit die Aktivierung der Aerosol-Erzeugung durch Übermittlung des Aktivierungssignals veranlassen kann.

In einer bevorzugten Ausführungsform weist die Aerosol-Erzeugungsvorrichtung eine zweite Steuereinheit auf, die eingerichtet ist zum Auslösen der Aerosol-Erzeugung durch den Zerstäuber bei Empfang des Aktivierungssignals. Beispielsweise weist die zweite Steuereinheit einen Empfänger zum Empfangen des Aktivierungssignals der Aktivierungsvorrichtung auf, wie etwa einen Funkempfänger oder IR-Empfänger. Bei einigen Ausführungsformen weist die zweite Steuereinheit einen elektronischen Schaltkreis wie etwa einen Microcontroller auf, der zur Auslösung der Aerosol-Erzeugung durch den Zerstäuber bei Empfang des Aktivierungssignals eingerichtet ist.

Die Aktivierungsvorrichtung und die Aerosol-Erzeugungsvorrichtung können drahtlos, insbesondere über Funk, oder alternativ über ein Kabel miteinander verbunden sein. Falls ein Kabel vorgesehen ist, ist das Aktivierungssignal bspw. in Form eines elektrischen Signals an die Aerosol-Erzeugungsvorrichtung übertragbar. Die zweite Steuereinheit ist vorzugsweise, aber nicht notwendigerweise in der Aerosol-Erzeugungsvorrichtung vorhanden. Beispielsweise ist das Aktivierungssignal direkt an den Zerstäuber zum Auslösen der Aerosol-Erzeugung übermittelbar, bspw. über ein Kabel.

Gemäß den hierin beschriebenen Ausführungsformen kann mittels der ersten Steuereinheit eine äußerst zuverlässige Aktivierung der Aerosol-Erzeugung als Reaktion auf das Ausatmen der Person in die Einblasöffnung erfolgen. Ferner kann das Aktivierungssignal optional nicht nur die Aktivierung der Aerosol-Erzeugung auslösen, sondern noch weitere Informationen zur nasalen Verabreichung an die Aerosol-Erzeugungsvorrichtung übermitteln, bspw. Informationen hinsichtlich Dosierung, Aerosol-Temperatur, Aerosol-Flussrate, Aerosol-Abgabedauer o. dgl. Das ermöglicht einen flexiblen Einsatz des Geräts und eine Anpassung an verschiedene Anwendungen.

Gemäß hierin beschriebenen Ausführungsformen wirkt die Aktivierungsvorrichtung als kabelgebundene oder drahtlose "Fernbedienung" für die Aerosol-Erzeugungsvorrichtung, und die Aktivierung der Aerosol-Erzeugung erfolgt mittels des Aktivierungssignals. Dies ermöglicht eine komfortable Handhabung des Geräts, weil eine Exhalation durch den Verwender in die Einblasöffnung der Aktivierungsvorrichtung erfolgen kann, während die Aerosol-Erzeugungsvorrichtung separat von der Aktivierungsvorrichtung zur Verabreichung des Aerosols an den Nasenraum an die Nase des Verwenders gehalten werden kann. Trotz der separaten Handhabbarkeit von Aerosol-Erzeugungsvorrichtung und Aktivierungsvorrichtung kann die Aktivierung der Aerosol-Erzeugung mittels des Aktivierungssignal stets zuverlässig erfolgen.

Eine Aktivierungsvorrichtung eines Geräts zur Verabreichung eines Aerosols an den Nasenraum ist hierin auch separat beschrieben und beansprucht. Eine solche Aktivierungsvorrichtung umfasst eine Einblasöffnung, in die eine Person ausatmen kann, eine erste Steuereinheit, die eingerichtet ist zur Übermittlung eines Aktivierungssignals an eine Aerosol-Erzeugungsvorrichtung als Reaktion auf ein Ausatmen einer Person in die Einblasöffnung, und einen Blaswiderstand, der dazu eingerichtet ist, dem Ausatmen der Person in die Einblasöffnung einen Widerstand entgegenzusetzen. Die Aktivierungsvorrichtung kann ferner beliebige Merkmale der Aktivierungsvorrichtung des hierin und im Folgenden beschriebenen Geräts aufweisen, insbesondere einen Sensor, eine Anzeige, ein Mundstück, ein erstes Verbindungsteil, einen Aufnahmebereich zur Aufnahme eines ersten Energiespeichers und/oder einen Haltebereich, usw.

Ferner ist hierin auch eine Aerosol-Erzeugungsvorrichtung eines Geräts zur Verabreichung eines Aerosols an den Nasenraum separat beschrieben und beansprucht. Die Aerosol-Erzeugungsvorrichtung umfasst einen Behälter zur Aufnahme einer Substanz, insbesondere eines Medikaments, einen Zerstäuber zur Überführung der Substanz in ein Aerosol und eine Ausgabeöffnung, durch die das Aerosol an den Nasenraum einer Person verabreichbar ist. Die Aerosol-Erzeugungsvorrichtung umfasst ferner eine zweite Steuereinheit, die eingerichtet ist zum Auslösen einer Aerosol-Erzeugung durch den Zerstäuber bei Empfang eines Aktivierungssignals von einer Aktivierungsvorrichtung, insbesondere von der hierin beschriebenen Aktivierungsvorrichtung. Die Aerosol-Erzeugungsvorrichtung kann ferner beliebige Merkmale der Aerosol-Erzeugungsvorrichtung des hierin und im Folgenden beschriebenen Geräts aufweisen, insbesondere einen Aufnahmebereich zur Aufnahme eines zweiten Energiespeichers, eine Ausgabeöffnung mit Nasen-Eingriffsstück, ein zweites Verbindungsteil und/oder eine Anzeige, usw.

Gemäß einigen Ausführungsformen, die mit anderen Ausführungsformen kombiniert werden können, sind die Aktivierungsvorrichtung und die Aerosol-Erzeugungsvorrichtung bei Verwendung nicht aneinander angebracht. Insbesondere können die Aktivierungsvorrichtung und die Aerosol-Erzeugungsvorrichtung körperlich getrennt voneinander angeordnet werden. So können die Aktivierungsvorrichtung und die Aerosol-Erzeugungsvorrichtung zur Verwendung des Geräts bspw. mit zwei verschiedenen Händen gehalten werden und separat voneinander an den Mund bzw. an die Nase geführt werden, was die Handhabung des Geräts vereinfacht. Bei alternativen Ausführungsformen können die Aktivierungsvorrichtung und die Aerosol-Erzeugungsvorrichtung auch bei Verwendung, also bei Exhalation in die Einblasöffnung, miteinander verbunden sein, bspw. aneinander angebracht sein.

Optional sind die Aktivierungsvorrichtung und die Aerosol-Erzeugungsvorrichtung lösbar aneinander anbringbar, bspw. mittels entsprechender Verbindungsmittel, die an der Aktivierungsvorrichtung und/oder der Aerosol-Erzeugungsvorrichtung vorgesehen sind. Das Gerät kann dann beispielsweise im Ruhezustand in "einstückiger Form" gehalten oder verstaut werden, wenn die Aktivierungsvorrichtung und die Aerosol-Erzeugungsvorrichtung aneinander angebracht sind. Hierdurch kann die Handhabbarkeit des Geräts weiter verbessert werden.

Vorzugsweise weist zu diesem Zweck die Aktivierungsvorrichtung ein erstes Verbindungsteil zum lösbaren Anbringen an der Aerosol-Erzeugungsvorrichtung auf. Alternativ oder zusätzlich weist zu diesem Zweck die Aerosol-Erzeugungsvorrichtung ein zweites Verbindungsteil zum lösbaren Anbringen an der Aktivierungsvorrichtung auf. Das erste Verbindungsteil kann mindestens ein magnetisches Element aufweisen, das zum Ankoppeln an mindestens ein zweites magnetisches Element der Aerosol-Erzeugungsvorrichtung eingerichtet ist. Das erste und/oder das zweite magnetische Element kann ferromagnetisch sein. Zumindest eines der magnetischen Elemente kann ein Permanentmagnet sein. Die Aktivierungsvorrichtung und die Aerosol-Erzeugungsvorrichtung können auf diese Weise schnell und einfach aneinander angebracht werden und wieder voneinander gelöst werden.

Gemäß einer besonders bevorzugten Ausführungsform ist die Aktivierungsvorrichtung zum Schließen des Gaumensegels (des Oropharyngealvelums) der in die Einblasöffnung ausatmenden Person eingerichtet. Mit anderen Worten verursacht die Aktivierungsvorrichtung das Schließen des Gaumensegels einer Person, wenn diese Person in die Einblasöffnung exhaliert.

Der Nasenraum umfasst insbesondere den nasalen Atemweg mit den durch die Nasenscheidewand getrennten Nasenhöhlen und zahlreichen Ostien, bspw. Nasennebenhöhlen und Tubenostien, und ist mit der Nasenschleimhaut überzogen. Der Nasenraum kann ferner den Nasenrachenraum, die eustachischen Röhren und die Paukenhöhlen umfassen. Der nasale Atemweg steht mit dem Nasenrachenraum, der Mundhöhle und dem unteren Atemweg in Verbindung, wobei das Gaumensegel im Wesentlichen am Übergang zwischen dem nasalen Atemweg und der Mundhöhle bzw. am Übergang zwischen dem Nasenrachen und dem Mundrachen angeordnet ist. Das Gaumensegel gibt in einer offenen Stellung eine Verbindung zwischen Nasenraum und Mundraum frei und verschließt diese Verbindung in einer geschlossenen Stellung. Die geschlossene Stellung kann durch Herbeiführen eines vorgegebenen Überdrucks in der Mundhöhle gegenüber dem Nasenraum herbeigeführt werden, wie er beispielsweise beim Ausatmen durch die Mundhöhle entstehen kann. Ein Ausatmen der Person in die Aktivierungsvorrichtung, insbesondere gegen den Blaswiderstand, kann deshalb gemäß den hierin beschriebenen Ausführungsformen zum Schließen des Gaumensegels führen.

Insbesondere wird das Schließen des Gaumensegels indirekt durch Erzeugen eines Überdrucks in der Mundhöhle gegenüber dem Nasenraum durch Ausatmen in die Aktivierungsvorrichtung bewirkt. Das Schließen des Gaumensegels durch das Ausatmen erfolgt dabei vorzugsweise gleichzeitig oder kurz vor der Auslösung der Aerosol-Erzeugung über das Aktivierungssignal, also vor der Verabreichung des Aerosols an den Nasenraum, durch Druckaufbau im Mundraum. Hierdurch wird sichergestellt, dass das Aerosol, das ein Medikament zur lokalen Verabreichung an den Nasenraum enthalten kann, größtenteils oder vollständig tatsächlich an den Nasenraum verabreicht wird und nicht oder kaum in den Mundraum vordringt.

Bei bevorzugten Ausführungsformen ist der Blaswiderstand eingerichtet zum Erzeugen des intra-oralen Überdrucks bei der in die Einblasöffnung ausatmenden Person, der zum Schließen des Gaumensegels führt. Beispielsweise umfasst die Aktivierungsvorrichtung einen sich an die Einblasöffnung anschließenden Hohlraum, bspw. in Form eines Rohrabschnitts, in den die Person ausatmen kann, wobei der Blaswiderstand in dem Rohrabschnitt angeordnet sein kann. Der Blaswiderstand kann als Strömungswiderstand wirken und der Person beim Ausatmen einen Widerstand entgegensetzen, so dass die Person gegen den Blaswiderstand ausatmet, wodurch der intra-orale Druck der Person beim Ausatmen in die Aktivierungsvorrichtung erhöht wird. Der Blaswiderstand kann beispielsweise als eine Verengung, eine Klappe, eine Membran, ein elastischer Ballon, eine Prallplatte, eine Ablenkplatte o. dgl. in einem Rohrabschnitt ausgebildet sein, der sich an die Einblasöffnung anschließt. Beispielsweise kann der Blaswiderstand eine Mehrzahl von Ablenkplatten in einem Rohrabschnitt aufweisen. Der Blaswiderstand kann im Inneren eines in der Aktivierungsvorrichtung ausgebildeten Hohlraums angeordnet sein. Der Blaswiderstand kann stationär (bspw. als Verengung eines Rohrabschnitt) oder beweglich (bspw. als in eine Schließstellung vorgespannte Klappe) ausgebildet sein. Der Blaswiderstand kann einstellbar sein, um eine Steuerung des beim Ausatmen erzeugten intra-oralen Drucks zu ermöglichen.

Gemäß einigen Ausführungsformen weist die Aktivierungsvorrichtung ein die Einblasöffnung ausbildendes Mundstück auf, durch das die Person in die Einblasöffnung ausatmen kann. Das Mundstück kann mit einem Grundkörper der Aktivierungsvorrichtung lösbar oder fest verbunden sein. Bspw. kann das Mundstück nach mehrmaligem Gebrauch ausgetauscht oder gereinigt werden und zu diesem Zweck abgenommen werden. Das Mundstück kann als rohrförmiger Abschnitt ausgebildet sein, der ein Durchblasen ermöglicht. Der Blaswiderstand kann innerhalb des Mundstücks angeordnet sein.

Vorzugsweise ist das Mundstück um eine Längsachse L drehbar an dem Grundkörper der Aktivierungsvorrichtung angeordnet. Zweckmäßigerweise weist die Aktivierungsvorrichtung ein Freigabeelement wie etwa einen Knopf oder eine Taste auf, das betätigbar ist, um eine Verdrehung des Mundstücks relativ zu dem Grundkörper freizugeben. Das Mundstück kann um einen vorgegebenen Winkel wie etwa bspw. 90° relativ zu dem Grundkörper verdreht werden, um die Aktivierungsvorrichtung zu aktivieren bzw. "einzuschalten", so dass die Aktivierungsvorrichtung zur Übermittlung des Aktivierungssignals bei Detektion einer Exhalation bereitsteht. Dies kann zu einer verbesserten Handhabung der Aktivierungsvorrichtung führen. Ein Verdrehen des Mundstücks zurück in die Ausgangsstellung kann die Aktivierungsvorrichtung wieder deaktivieren bzw. "ausschalten". Andere Einschalt- bzw. Ausschaltmechanismen sind ebenfalls vorstellbar.

Die Aktivierungsvorrichtung kann ferner einen Sensor zum Erfassen einer Exhalation in die Einblasöffnung aufweisen, wie etwa einen Drucksensor oder einen Strömungssensor. Insbesondere weist die Aktivierungsvorrichtung einen Drucksensor auf, der eingerichtet ist zum Erfassen eines Drucks oder einer Druckänderung in der Aktivierungsvorrichtung, bspw. im Inneren des Mundstücks bzw. des dadurch ausgebildeten Rohrabschnitts. Der Drucksensor kann im Inneren eines in der Aktivierungsvorrichtung ausgebildeten Hohlraums stromaufwärts des Blaswiderstands angeordnet sein. Ein von dem Drucksensor gemessener Druck oder eine Druckänderung kann an die erste Steuereinheit übermittelt werden. Falls der Druck oder die Druckänderung einen vorgegebenen Wert übersteigt (so dass bspw. zuverlässig ein Schließen des Gaumensegels sichergestellt ist), kann die erste Steuereinheit die Übermittlung des Aktivierungssignals an die Aerosol-Erzeugungsvorrichtung veranlassen bzw. durchführen.

Die Aktivierungsvorrichtung kann ferner eine Anzeige aufweisen, die dazu eingerichtet ist, einen Operationszustand der Aktivierungsvorrichtung anzuzeigen. Beispielsweise wird die Anzeige aktiviert bei Erreichen eines vorgegebenen Drucks beim Ausatmen der Person in die Einblasöffnung, der zum Auslösen der Aerosol-Erzeugung führt. Die Anzeige kann das Erreichen des vorgegebenen Drucks visuell, akustisch und/oder taktil anzeigen. Beispielsweise kann die Anzeige eine Leuchtanzeige wie etwa eine LED, eine akustische Anzeige, die einen Ton oder ein anderes Audiosignal ausgibt, und/oder einen Vibrationsgenerator aufweisen.

Alternativ oder zusätzlich kann die Aerosol-Erzeugungsvorrichtung eine Anzeige aufweisen, die dazu eingerichtet ist, einen Operationszustand der Aerosol-Erzeugungsvorrichtung anzuzeigen, bspw. eine visuelle, akustische und/oder taktile Anzeige. Beispielsweise kann die Anzeige während der Verabreichung des Aerosols aktiviert sein, und/oder sie kann deaktiviert sein, wenn kein Aerosol verabreicht wird.

Zweckmäßigerweise kann die Aktivierungsvorrichtung eingerichtet sein zur Aufnahme eines ersten Energiespeichers, insbesondere einer Batterie, zum Bereitstellen von Energie für die Aktivierungsvorrichtung. Beispielsweise weist die Aktivierungsvorrichtung ein Batterie-Aufnahmefach zur Aufnahme von einer oder mehreren Batterien wie etwa Knopfzellen auf. Der erste Energiespeicher kann die Energie für den Betrieb der ersten Steuereinheit, des Drucksensors, der Anzeige und/oder zum Versenden des Aktivierungssignals, bspw. über eine Funk-Sendeeinheit, bereitstellen. Bei einigen Ausführungsformen ist ein erster Energiespeicher jedoch nicht vorgesehen, bspw. wenn die erste Steuereinheit einen passiven Transponder aufweist, der bspw. durch ein von der Aerosol-Erzeugungsvorrichtung ausgehendes Feld gespeist werden kann. Ein erster Energiespeicher ist auch dann nicht notwendigerweise erforderlich, wenn die Aktivierungsvorrichtung und die Aerosol-Erzeugungsvorrichtung über ein Kabel verbunden sind, wobei über das Kabel neben dem Aktivierungssignal auch elektrische Energie übertragbar ist.

Alternativ oder zusätzlich ist die Aerosol-Erzeugungsvorrichtung eingerichtet zur Aufnahme eines zweiten Energiespeichers, insbesondere einer Batterie, zum Bereitstellen von Energie für die Aerosol-Erzeugung und/oder deren Auslösung. Beispielsweise weist die Aerosol-Erzeugungsvorrichtung ein Batterie-Aufnahmefach zur Aufnahme von einer oder mehreren Batterien wie etwa Knopfzellen auf. Der zweite Energiespeicher kann die Energie für den Betrieb des Zerstäubers, der Anzeige, der zweiten Steuereinheit, zum Empfangen des Aktivierungssignals und/oder zum Auslösen der Aerosol-Erzeugung bereitstellen. Bei einigen Ausführungsformen ist ein zweiter Energiespeicher jedoch nicht vorgesehen, bspw. wenn der Zerstäuber ein unter Druck stehendes Gas als Energiequelle hat und/oder wenn eine für den Betrieb der Aerosol-Erzeugungsvorrichtung erforderliche Energie über die Aktivierungsvorrichtung drahtlos oder kabelgebunden bereitgestellt werden kann.

Die Handhabbarkeit der Aktivierungsvorrichtung kann verbessert werden, wenn die Aktivierungsvorrichtung zumindest einen Haltebereich zum Ergreifen und Halten der Aktivierungsvorrichtung mit einer Hand oder einem Teil davon bei Verwendung aufweist. Der zumindest eine Haltebereich kann zumindest eine Vertiefung aufweisen, die an den Eingriff eines Fingers angepasst sein kann, so dass die Aktivierungsvorrichtung bequem mit einer Hand gehalten werden kann. Beispielsweise können an einem Grundkörper der Aktivierungsvorrichtung zwei in entgegengesetzte Richtungen weisende Haltevertiefungen zum Eingriff von Daumen und Zeigefinger vorgesehen sein.

Die Aerosol-Erzeugungsvorrichtung weist vorzugsweise neben dem Zerstäuber einen zur Aufnahme einer Substanz eingerichteten Behälter auf, wobei der Zerstäuber zur Überführung der Substanz in das Aerosol eingerichtet ist. Die Substanz kann einen oder mehrere der folgenden Bestandteile aufweisen: ein oder mehrere Medikamente, ein Reinigungsmittel zum Reinigen des Nasenraums, einen medikamentenhaltigen Feststoff wie etwa ein Pulver oder eine medikamentenhaltige Flüssigkeit. Bei der Behandlung von Erkrankungen der Nase kann eine örtliche (lokale) Medikamentenverabreichung an den Nasenraum zu bevorzugen sein (bspw. von Kortison wie z.B. Mometasonfuroat). Alternativ oder zusätzlich kann aber auch eine derartige Medikamentenverabreichung an den Nasenraum zu bevorzugen sein, um eine systemische Wirkung zu erzielen, bspw. zur Prophylaxe und/oder Behandlung von Erkrankungen, wie beispielsweise Osteoporose, Migräne, als Hormonersatztherapie (e.g., Insulingabe), oder als Impfung. Der Behälter kann an die zu verabreichende Substanz angepasst sein, bspw. an die Aufnahme eines Pulvers und/oder einer Flüssigkeit und/oder an eine abzugebende Menge der Substanz für eine oder mehrere Anwendungen.

Die Aerosol-Erzeugungsvorrichtung weist ferner neben dem Zerstäuber vorzugsweise eine Ausgabeöffnung auf, durch die das Aerosol an den Nasenraum der Person verabreichbar ist. Vorzugsweise weist die Aerosol-Erzeugungsvorrichtung ein die Ausgabeöffnung ausbildendes Nasen-Eingriffsstück auf, das optional um eine Drehachse drehbar an der Aerosol-Erzeugungsvorrichtung angeordnet sein kann. Das Nasen-Eingriffsstück kann abnehmbar an der Aerosol-Erzeugungsvorrichtung angebracht sein, so dass es zur Reinigung oder zum Wechseln nach mehrmaligem Gebrauch abgenommen werden kann. Außerdem lässt sich so ein an eine bestimmte Nasengröße angepasstes Nasen-Eingriffsstück verwenden. Das Nasen-Eingriffsstück kann bspw. über eine Schnappverbindung an einem vorstehenden Ende eines Aerosol-Ausgabekanals lösbar angebracht sein.

Das Nasen-Eingriffsstück kann zum Eingriff in ein Nasenloch des Verwenders eingerichtet sein und eine Durchgangsöffnung aufweisen, durch die das Aerosol in das Nasenloch einströmen kann. Das Nasen-Eingriffsstück kann zur Erzielung einer flüssigkeitsdichten Abdichtung zum Nasenloch hin ausgebildet sein. Beispielsweise kann das Nasen-Eingriffsstück aus einem elastischen Material wie etwa einem elastischen Polymer oder Silikon ausgebildet sein. Das Nasen-Eingriffsstück kann eine umlaufende Schrägfläche aufweisen, so dass es mit Nasenlöchern unterschiedlicher Größe dichtend in Eingriff bringbar ist. Optional können auch zwei Nasen-Eingriffsstücke vorgesehen sein, die zwei Ausgabeöffnungen zur gleichzeigen Abgabe des Aerosols in beide Nasenlöcher ausbilden.

Vorzugsweise ist das Nasen-Eingriffsstück so eingerichtet, dass es mehr als 1 mm und weniger als 2 cm in ein Nasenloch hineinragt, wenn es zur Verabreichung des Aerosols mit dem Nasenloch in Eingriff gebracht ist. Die Form des Nasen-Eingriffsstücks kann an bestimmte Bedürfnisse angepasst sein. Beispielsweise kann das Nasen-Eingriffsstück derart geformt sein, dass eine Turbulenzbildung und/oder eine gute Dispersion des Aerosols gefördert wird. Das Nasen-Eingriffsstück kann insbesondere eine Nasenolive aufweisen, die zur Bildung eines geeigneten physiologischen Gasstroms beitragen kann.

Die Aerosol-Erzeugungseinrichtung kann einen Sensor aufweisen, der dazu eingerichtet ist, eine Position und/oder Lage der Aerosol-Erzeugungseinrichtung zu bestimmen, insbesondere in Bezug zu der Nase bzw. der Nasenöffnung des Verwenders. Beispielsweise kann der Sensor dazu eingerichtet sein, eine Position und/oder Lage des Nasen-Eingriffsstücks der Aerosol-Erzeugungsvorrichtung zu messen, insbesondere in Bezug zu der Nase bzw. Nasenöffnung des Verwenders, in die das Nasenstück zur Aerosolabgabe hineinragt.

In einigen Ausführungsformen kann eine Anzeige vorgesehen sein, die eingerichtet ist zum Erzeugen eines (bspw. akustischen optischen und/oder taktilen) Anzeigesignals in Abhängigkeit von einem Messsignal des Sensors. Beispielsweise kann die Anzeige zum Erzeugen eines (ersten) Anzeigesignals eingerichtet sein, wenn die Aerosol-Erzeugungseinrichtung oder deren Nasen-Eingriffsstück eine vorgegebene Position und/oder Lage einnimmt, insbesondere in Bezug zu der Nase bzw. der Nasenöffnung des Verwenders. Der Verwender kann durch das (erste) Anzeigesignal erkennen, dass er die Aerosol-Erzeugungseinrichtung gerade in einer korrekten Lage hält und kann dann durch Ausatmen in die Einblasöffnung die Aerosol-Erzeugung auslösen. Alternativ oder zusätzlich kann die Anzeige zum Erzeugen eines (zweiten) Anzeigesignals dann eingerichtet sein, wenn die Aerosol-Erzeugungseinrichtung oder deren Nasen-Eingriffsstück nicht in einer vorgegebenen Position und/oder Lage angeordnet ist. Der Verwender kann durch das (zweite) Anzeigesignal erkennen, dass er die Aerosol-Erzeugungseinrichtung nicht in einer korrekten Lage hält und kann deren Lage korrigieren, bspw. durch Verschieben, Kippen oder Umpositionieren des Nasen-Eingriffsstücks in der Nasenöffnung, bevor er die Aerosolerzeugung auslöst.

Alternativ oder zusätzlich kann eine Steuereinheit der Aerosol-Erzeugungseinrichtung (insbesondere die zweite Steuereinheit) derart eingerichtet sein, dass sie die Aerosol-Erzeugung durch den Zerstäuber oder die Verabreichung des Aerosols an den Nasenraum in Abhängigkeit von einem Messsignal des Sensors auslöst.

Beispielsweise ist die Steuereinheit derart eingerichtet, dass sie die Aerosol-Erzeugung oder die Verabreichung des Aerosols nur dann auslöst, wenn die Aerosol-Erzeugungseinrichtung oder deren Nasen-Eingriffsstück eine vorgegebene Position, und/oder Lage einnimmt, insbesondere in Bezug zu der Nase bzw. der Nasenöffnung des Verwenders. Zu diesem Zweck kann ein Messsignal des Sensors an die Steuereinheit übermittelt werden, so dass die Steuereinheit die Aerosolerzeugung in Abhängigkeit von dem Sensorsignal auslösen kann. Auf diese Weise kann eine Fehlabgabe des Aerosols in Fällen verhindert werden, in denen der Verwender die Aerosol-Erzeugungseinrichtung in einer falschen Position und/oder Lage an seine Nase hält. Insbesondere kann die Wahrscheinlichkeit eines fehlgeleiteten (bspw. an der Nase vorbei) oder in seiner Wirkung verminderten (bspw. durch schlechten Strömungswinkel) Aerosolstroms verringert werden.

Der Sensor kann beispielsweise einen Abstands- oder Entfernungssensor aufweisen, bspw. einen Laser- oder LED-Entfernungsmesser oder einen nicht-optischen oder optischen Näherungsschalter wie etwa einen IR-Distanzsensor. Der Sensor kann eine freie Weglänge ausgehend von der Aerosol-Erzeugungseinrichtung bestimmen. In einigen Ausführungsformen kann der Sensor im Bereich des Nasen-Eingriffsstücks angeordnet sein, zum Beispiel an oder in der Aerosol-Abgabeöffnung des Geräts, vorzugsweise derart, dass er bei korrekter Positionierung der Aerosol-Erzeugungseinrichtung geradewegs in die Nasenhöhle "hineinleuchtet". Eine korrekte Positionierung kann durch einen Messwert des Sensors innerhalb eines vorgegebenen Wertebereichs erkennbar sein. Beispielsweise kann ein Messwert des Sensors unterhalb eines unteren Schwellenwerts auf einen fehlerhaften Einführwinkel des Nasen-Eingriffsstücks in die Nasenöffnung hindeuten, und/oder ein Messwert des Sensors oberhalb eines oberen Schwellenwerts kann darauf hindeuten, dass das Nasen-Eingriffsstück noch gar nicht in die Nasenöffnung hineinragt oder vollkommen falsch platziert ist.

Alternativ oder zusätzlich kann die Aerosol-Erzeugungsrichtung einen Lagesensor (bspw. ein Magnetometer oder Gyroskop) zum Bestimmen einer Lage bzw. Orientierung der Aerosol-Erzeugungseinrichtung umfassen. Über den Messwert des Lagesensors kann bspw. erkannt werden, ob sich die Aerosol-Erzeugungseinrichtung etwa in einer korrekten Lage (bspw. unter einem im Hinblick auf die Aerosolabgabe vorteilhaften Winkel relativ zur Vertikalen, der im Bereich des Verlaufswinkels typischer Nasenhöhlen relativ zur Vertikalen liegt, o. dgl.) und/oder in einer vollkommen falschen Lage (bspw. horizontal anstelle von vertikal, o.dgl.) befindet.

Auch andere Arten von Sensoren zur Lage und/oder Positionsbestimmung sind vorstellbar, wie etwa eine Kamera, ein Drucksensor am Nasen-Eingriffsstück, o. dgl.

Bei einigen Ausführungsformen, die mit anderen hierin beschriebenen Ausführungsformen kombiniert werden können, umfasst die Aerosol-Erzeugungsvorrichtung eine erste Untereinheit und eine mit der ersten Untereinheit lösbar verbundene zweite Untereinheit. Die erste Untereinheit weist den Zerstäuber, den Behälter, die Ausgabeöffnung (bzw. einen Aerosol-Ausgabekanal mit der Ausgabeöffnung) auf, und die zweite Untereinheit weist die zweite Steuereinheit und/oder ein Batterie-Aufnahmefach auf. Die erste Untereinheit kann von der zweiten Untereinheit abgenommen werden, bspw. um Batterien zu wechseln oder um eine Substanz im Behälter nachzufüllen. Die erste Untereinheit kann über Verbindungselemente mit der zweiten Untereinheit verbunden sein. Bspw. kann die erste Untereinheit magnetisch an die zweite Untereinheit koppelbar sein. Jede der Untereinheiten kann einen Gehäuseabschnitt aufweisen. Alternativ kann die Aerosol-Erzeugungsvorrichtung auch ein Einteil-Gehäuse aufweisen, dass keine Abnahme einer separierbaren Untereinheit ermöglicht.

Bei einigen Ausführungsformen, die mit anderen hierin beschriebenen Ausführungsformen kombiniert werden können, kann der Aerosol-Ausgabekanal um eine Längsachse L drehbar vom Rest (d.h. vom Grundkörper) der ersten Untereinheit angeordnet sein. Zweckmäßigerweise weist die erste Untereinheit ein Freigabeelement wie etwa einen Knopf oder eine Taste auf, das betätigbar ist, um eine Verdrehung des Aerosol-Ausgabekanals relativ zu dem Grundkörper freizugeben. Der Aerosol-Ausgabekanal kann um einen vorgegebenen Winkel wie etwa bspw. 90° oder 180° relativ zu dem Grundkörper verdreht werden. Dies kann zu einer verbesserten Handhabung der Aerosol-Erzeugungsvorrichtung führen, da die Zugänglichkeit zur Nase erleichtert wird.

In einer bevorzugten Ausführungsform ist die Ausgabeöffnung angrenzend an die Aktivierungsvorrichtung angeordnet, wenn die Aktivierungsvorrichtung und die Aerosol-Erzeugungsvorrichtung aneinander angebracht sind. Beispielsweise ragt der die Ausgabeöffnung aufweisende Aerosol-Ausgabekanal ausgehend von dem Zerstäuber in eine Richtung vor, in der die Aktivierungsvorrichtung an der Aerosol-Erzeugungsvorrichtung angebracht ist. Auf diese Weise kann ein kompaktes und handliches Gerät bereitgestellt werden.

Vorzugsweise weist die Aerosol-Erzeugungsvorrichtung eine Aerosol-Abgabesteuerung und/oder einen Luftstrombeschleuniger zum Bereitstellen einer vorgegebenen Aerosol-Flussrate und/oder Aerosol-Flussgeschwindigkeit auf, die durch die Ausgabeöffnung ausgegeben wird. Die Aerosol-Abgabesteuerung kann optional zum Beibehalten der vorgegebenen Aerosol-Flussrate über einen vorgegebenen Zeitraum eingerichtet sein. Der Luftstrombeschleuniger, bspw. ein Ventilator oder eine Mikropumpe, kann eine Einströmung des Aerosols in den Nasenraum mit einer vorgegebenen Geschwindigkeit und/oder einer vorgegebenen Rate bereitstellen. Die Aerosol-Abgabesteuerung kann die von dem Zerstäuber pro Zeiteinheit bzw. über einen vorgegebenen Zeitraum zu erzeugende Aerosolmenge gewährleisten, also bspw. eine Aerosol-Flussrate und/oder eine insgesamt abzugebende Aerosolmenge. Optional kann das Aktivierungssignal eine Aerosol-Abgabeinformation enthalten, die die Aerosol-Abgabe beeinflusst, bspw. durch entsprechende Einstellung der Aerosol-Abgabesteuerung und/oder des Luftstrombeschleunigers.

Bei einigen Ausführungsformen, die mit anderen hierin beschriebenen Ausführungsformen kombiniert werden können, ist der Luftstrombeschleuniger eine Mikropumpe. Beispielhafte Mikropumpen können peristaltische Systeme sein. Peristaltische Systeme sind eine Nachahmung der Natur. Vor allem bei nichtmechanischen Pumpen ergeben sich große Vorteile durch Miniaturisierung. So können durch die kleinen Geometrien sehr hohe elektrische bzw. magnetische Feldstärken erreicht werden. Der Effekt - elektrokinetisch, elektrohydrodynamisch, elektrohydromagnetisch, elektrochemisch etc. - ist um ein Vielfaches wirkungsvoller als in der Makrowelt. Vorzugsweise kann die hierin verwendbare Mikropumpe eine Piezomembran enthalten.

Bei einigen Ausführungsformen, die mit anderen hierin beschriebenen Ausführungsformen kombiniert werden können, ist der Luftstrombeschleuniger ein Ventilator. Beispielhafte Ventilatoren können Axialventilator, Diagonalventilator, Radial-/Zentrifugalventilator, oder Tangential- bzw. Querstromventilatoren sein.

In einer bevorzugten Ausführungsform ist die Aerosol-Erzeugungsvorrichtung dazu eingerichtet, eine Aerosol-Flussrate von 5 l/min oder mehr und 50 I/min oder weniger an den Nasenraum zu verabreichen, vorzugsweise 10 I/min oder mehr und 30 I/min oder weniger, insbesondere etwa 15 l/min.

Die Aerosol-Erzeugungsvorrichtung kann zumindest ein Heizelement zum Heizen des Aerosols auf eine vorgegebene Temperatur aufweisen. Dies ermöglicht die Abgabe des Aerosols an den Nasenraum unter einer vorgegebenen Abgabetemperatur.

Die Aerosol-Erzeugungsvorrichtung kann eine Fördereinrichtung wie etwa eine Mikropumpe aufweisen, die zur Zuführung einer vorgegebenen Menge der Substanz an den Zerstäuber bei Auslösung der Aerosol-Erzeugung eingerichtet ist.

Der Zerstäuber ist zum Überführen einer Substanz in das Aerosol eingerichtet. "Zerstäuben" wird hierin breit verstanden als Überführen einer Substanz (fluid oder solid) in ein Aerosol und ist nicht beschränkt auf das Versprühen einer Flüssigkeit. Unter anderem umfasst "Zerstäuben" also auch Verdampfen, Vernebeln, Sublimieren und Versprühen von Pulver. Die Überführung der Substanz in das Aerosol kann insbesondere durch einen oder mehrere der folgenden Mechanismen (1)-(6) erfolgen:
(1) Durch Heizen der Substanz mit einem Heizelement: Der Zerstäuber kann ein Heizelement wie etwa eine oder mehrere Heizwendel oder Heizspiralen (bspw. nach Art eines "Coils" einer E-Zigarette) zum Verdampfen der Substanz aufweisen. Eine vorgegebene Dosierung der Substanz kann zum Verdampfen in die Umgebung des Heizelements oder in Kontakt mit dem Heizelement gebracht werden, bspw. mittels einer Fördereinrichtung wie etwa einer Mikropumpe oder eines Dochts. Der generierte Dampf kann als Aerosol an den Nasenraum abgegeben werden, bspw. aufgrund Diffusion durch die Ausgabeöffnung oder zusätzlich mittels eines Luftstrombeschleunigers, wie etwa einer Mikropumpe oder eines Ventilators. Insbesondere kann der Zerstäuber nach Art eines Verdampferkopfs einer E-Zigarette eingerichtet sein.
(2) Durch Versetzen der Substanz in Schwingungen über ein Ultraschallelement: Der Zerstäuber kann ein Ultraschallelement wie etwa einen Ultraschallvernebler aufweisen, der bspw. ein in Schwingung versetzbares piezoelektrisches Element aufweisen kann. Eine vorgegebene Dosierung der Substanz kann durch Versetzen in Schwingungen vernebelt werden, bspw. indem sie auf ein in Schwingung versetzbares Plättchen des Ultraschallelements aufgebracht wird, insbesondere mittels eines Förderelements wie etwa einer Mikropumpe. Der generierte Dampf kann als Aerosol an den Nasenraum abgegeben werden, bspw. aufgrund Diffusion durch die Ausgabeöffnung oder zusätzlich mittels eines Luftstrombeschleunigers. Insbesondere kann der Zerstäuber nach Art eines Ultraschallverneblers einer E-Zigarette eingerichtet sein.
(3) Der Zerstäuber kann eine Vorrichtung zum Leiten eines komprimierten Gases durch die Substanz aufweisen. Die Substanz kann durch den Gasstrom mitgerissen werden und der die Substanz führende Gasstrom kann als Aerosol an den Nasenraum abgegeben werden. Beispielsweise weist der Zerstäuber eine unter Druck stehende Gasquelle zum Mitreißen einer abgemessenen Dosierung der Substanz auf, bspw. eines medikamentenhaltigen Pulvers.
(4) Mittels einer Schwingmembran: Der Zerstäuber kann eine Membran mit Öffnungen (bspw. Mikro-Öffnungen) aufweisen, die in Schwingungen versetzt werden kann. Eine auf einer Seite der Membran angeordnete flüssige Substanz benetzt die Membran, tritt durch die Öffnungen und verlässt diese auf der anderen Seite der Membran als Aerosol. Die Schwingmembran kann bspw. mittels eines piezoelektrischen Elements in Schwingungen versetzt werden.
(5) Der Zerstäuber kann eine Düse aufweisen, durch die die Substanz zum Generieren des Aerosols gesprüht wird, insbesondere unter Verwendung eines Treibmittels unter Druck.
(6) Der Zerstäuber kann ein Inhalationsgerät, wie etwa einen Hochdruckinhalator mit einer vorgegebenen Dosierung der Substanz aufweisen. Der Inhalator kann zum Verdampfen der Substanz zum Bereitstellen des Aerosols ausgebildet sein.

Der Zerstäubungsmechanismus ist nicht auf die obigen Beispiele beschränkt, sondern umfasst beliebige Mechanismen, die zur Überführung der Substanz in das Aerosol geeignet sind.

Die Aerosol-Erzeugungsvorrichtung weist vorzugsweise eine zweite Steuereinheit zum Auslösen der Aerosol-Erzeugung durch den Zerstäuber bei Empfang des Aktivierungssignals von der Aktivierungsvorrichtung auf. Das Aktivierungssignal kann die Erzeugung einer vorgegeben Aerosolmenge und/oder die Erzeugung des Aerosols über einen vorgegebenen Zeitraum auslösen. Alternativ oder zusätzlich kann das Aktivierungssignal übermittelt werden, solange die Person in die Einblasöffnung der Aktivierungsvorrichtung ausatmet, so dass die Aerosol-Erzeugung gestoppt werden kann, sobald die Person nicht mehr ausatmet und/oder wenn bereits eine vorgegebene Maximalmenge des Aerosols an den Nasenraum abgegeben ist. Alternativ kann ein Deaktivierungssignal von der Aktivierungsvorrichtung übermittelt werden, sobald die Person nicht mehr in die Einblasöffnung ausatmet, woraufhin die Aerosol-Erzeugung gestoppt werden kann.

Ein hierin beschriebenes Gerät kann verwendet werden zur Erzeugung eines Aerosols und zur Verabreichung des Aerosols an den Nasenraum einer Person, solange die Person in eine Einblasöffnung der Aktivierungsvorrichtung des Geräts ausatmet. Das Ausatmen in die Einblasöffnung führt zur Übermittlung eines Aktivierungssignals von der Aktivierungsvorrichtung an die Aerosol-Erzeugungsvorrichtung, das eine Aerosol-Erzeugung auslöst.

Ferner ist hierin in Verfahren zum Erzeugen eines Aerosols beschrieben. Das Verfahren umfasst das Ausatmen einer Person in eine Einblasöffnung einer Aktivierungsvorrichtung eines Geräts zur Verabreichung eines Aerosols. Das Verfahren umfasst ferner die Übermittlung eines Aktivierungssignals von der Aktivierungsvorrichtung an eine Aerosol-Erzeugungsvorrichtung des Geräts als Reaktion auf das Ausatmen.

Das Aktivierungssignal führt zu einer Aerosol-Erzeugung durch einen Zerstäuber der Aerosol-Erzeugungsvorrichtung und zum Verabreichen des Aerosols an den Nasenraum der Person, insbesondere über ein in die Nase der Person eingreifendes Nasen-Eingriffsstück.

Das Gerät ist insbesondere ein Gerät gemäß einer hierin beschriebenen Ausführungsform.

Vorzugsweise sind die Aerosol-Erzeugungsvorrichtung und die Aktivierungsvorrichtung beim Ausatmen der Person in die Einblasöffnung nicht aneinander angebracht.

Vorzugsweise erfolgt die Übermittlung des Aktivierungssignals drahtlos, bspw. über Funk, Infrarotstrahlung und/oder mittels eines Transponders. Alternativ kann auch eine kabelgebundene Übermittlung vorgesehen sein.

Vorzugsweise enthält das Aerosol ein Medikament.

In der nun folgenden Beschreibung wird die Erfindung unter Bezugnahme auf die beigefügten Zeichnungen erläutert. Darin zeigt:
FIG. 1 eine offenbarungsgemäße Aktivierungsvorrichtung;
FIG. 2 eine offenbarungsgemäße Aerosol-Erzeugungsvorrichtung;
FIG. 3 ein offenbarungsgemäße Gerät in einem gekoppelten Zustand; und
FIG. 4 ein offenbarungsgemäße Gerät bei Verwendung in einem separierten Zustand.

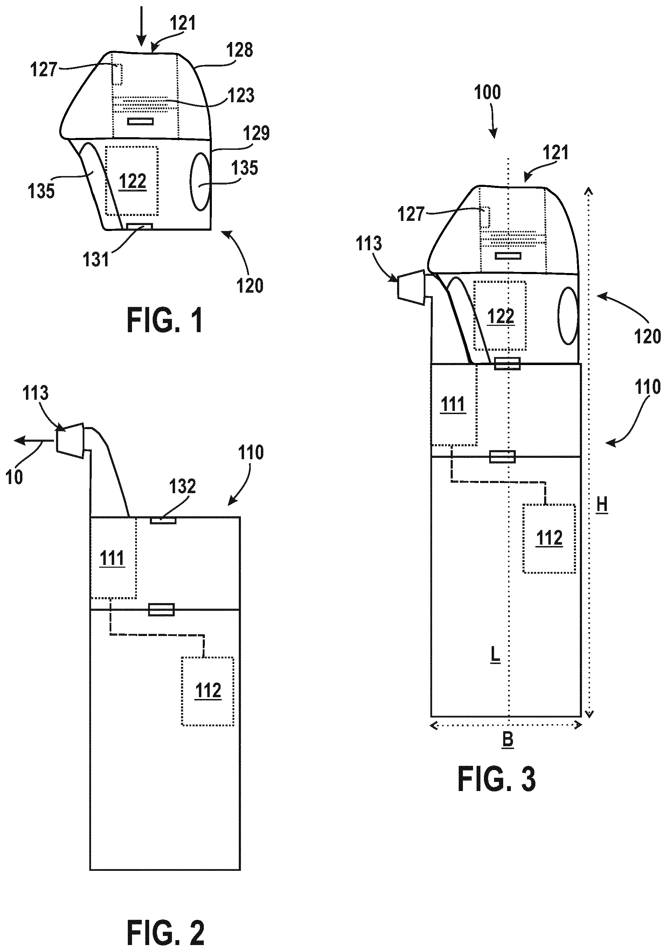

FIG. 1 ist eine schematische Darstellung einer offenbarungsgemäße Aktivierungsvorrichtung 120, die einen ersten Teil eines Geräts 100 zur Verabreichung eines Aerosols 10 an den Nasenraum darstellt. FIG. 2 ist eine schematische Darstellung einer erfindungsgemäßen Aerosol-Erzeugungsvorrichtung 110, die einen zweiten Teil des Geräts 100 darstellt. Das ganze Gerät 100 ist in FIG. 3 schematisch in einem gekoppelten Zustand dargestellt, in dem die Aktivierungsvorrichtung 120 und die Aerosol-Erzeugungsvorrichtung 110 aneinander angebracht sind.

Wie in FIG. 1 gezeigt ist, weist die Aktivierungsvorrichtung 120 eine Einblasöffnung 121 auf, in die eine Person ausatmen kann und die von einem Mundstück 128 ausgebildet ist. Das Mundstück 128 kann einen rohrförmigen Abschnitt (in FIG. 1 gestrichelt dargestellt) aufweisen, der ein Ausatmen der Person durch das Mundstück 128 ermöglicht.

Die Aktivierungsvorrichtung 120 weist ferner einen Blaswiderstand 123 auf, der in FIG. 1 beispielhaft als eine Mehrzahl von Ablenkplatten dargestellt ist. Der Blaswiderstand 123 kann innerhalb des rohrförmigen Abschnitts angeordnet sein, der von dem Mundstück 128 ausgebildet ist. Der Blaswiderstand 123 ist dazu eingerichtet, dem Ausatmen der Person einen Widerstand entgegenzusetzen, so dass sich beim Ausatmen der Person ein positiver intra-oraler Druck in der Mundhöhle der Person einstellt, der zum Schließen des Gaumensegels führt.

Das Mundstück 128 kann verdrehbar an einem Grundkörper 129 der Aktivierungsvorrichtung 120 angebracht sein. Die Verdrehbarkeit kann durch Betätigung eines Freigabeelement freigegeben werden, bspw. durch Druck auf eine Freigabetaste. Eine Verdrehung des Mundstücks 128 relativ zu dem Grundkörper 129 um einen vorgegebenen Winkel (bspw. um 90°) kann die Aktivierungsvorrichtung aktivieren bzw. anschalten. Eine Verdrehung des Mundstücks 128 zurück in die Ausgangsstellung kann die Aktivierungsvorrichtung deaktivieren bzw. ausschalten.

Die Aktivierungsvorrichtung 120 weist ferner eine erste Steuereinheit 122 auf, die eingerichtet ist zur Aktivierung der Aerosol-Erzeugungsvorrichtung 110 als Reaktion auf das Ausatmen der Person in die Einblasöffnung 121. Die erste Steuereinheit 122 kann dazu eingerichtet sein, ein Aktivierungssignal an die Aerosol-Erzeugungsvorrichtung zu übermitteln, wenn ein vorgegebener Druck oder eine vorgegebene Atemströmung in dem Mundstück 128 festgestellt werden, bspw. mittels eines Sensors, wie etwa eines Drucksensors 127 oder eines Strömungssensors.

Die erste Steuereinheit 122 kann zum drahtlosen oder zum kabelgebundenen Aktivieren der Aerosol-Erzeugungseinheit 110 eingerichtet sein, bspw. über Kabel oder mittels eines Funk- oder IR-Signals. Insbesondere weist die erste Steuereinheit 122 einen elektrischen Schaltkreis auf, wie etwa einen integrierten Schaltkreis (IC), Steuerchip oder Microcontroller, der dazu eingerichtet ist, das Aktivierungssignal an die Aerosol-Erzeugungsvorrichtung 110 zu übermitteln, wenn ein Ausatmen in die Aktivierungsvorrichtung 120 festgestellt wird. Das Aktivierungssignal kann drahtlos gesendet werden, bspw. mittels eines Senders mit Antenne oder einer IR-Diode, die Teil der ersten Steuereinheit 122 sein können.

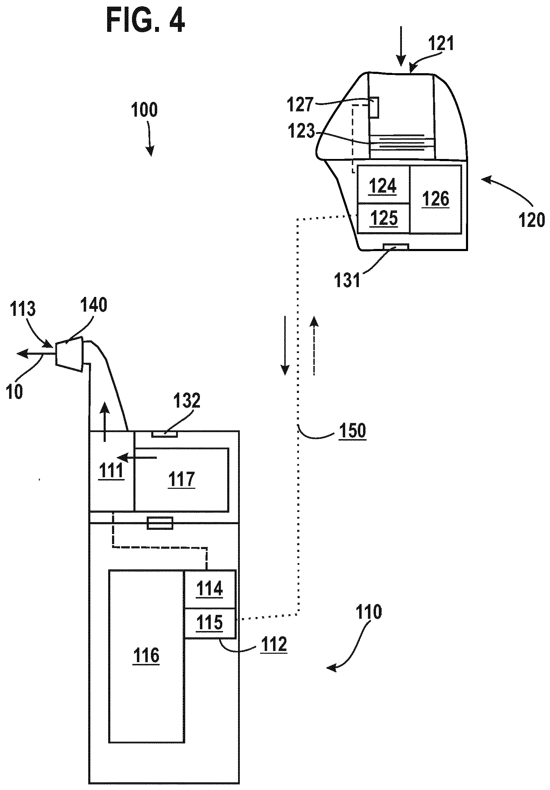

Wie in FIG. 4 dargestellt ist, kann die erste Steuereinheit einen integrierten Schaltkreis 124 mit Sender 125 zum drahtlosen Versenden des Aktivierungssignals 150 aufweisen. Optional kann die erste Steuereinheit einen Empfänger bzw. einen Transceiver aufweisen, so dass eine bidirektionale Kommunikation mit der Aerosol-Erzeugungsvorrichtung 110 ermöglicht ist. In einer möglichen Ausführungsform umfasst die erste Steuereinheit einen Transponder mit Microcontroller, bspw. einen passiven oder aktiven RFID-Transponder.

Das Aktivierungssignal 150 kann direkt oder indirekt (bspw. über WLAN) an eine zweite Steuereinheit 112 der Aerosol-Erzeugungsvorrichtung 110 übermittelt werden, die bspw. einen integrierten Schaltkreis 114 mit Empfänger 115 aufweisen kann (siehe FIG. 4), bspw. einen Funkempfänger oder eine Photodiode. Optional kann ein Sender bzw. ein Transceiver vorgesehen sein, so dass eine bidirektionale Kommunikation mit der Aktivierungsvorrichtung 120 ermöglicht ist. Alternativ kann das Aktivierungssignal 150 direkt an den Zerstäuber 111 zum Auslösen der Aerosol-Erzeugung übermittelt werden, bspw. über ein Kabel oder eine andere elektrische Verbindung.

Optional kann die erste Steuereinheit und/oder die zweite Steuereinheit einen Steuerchip mit Prozessor (bspw. mit einer CPU) und/oder einen Datenspeicher, bspw. einen Flash-Speicher, aufweisen. Optional ist die erste Steuereinheit und/oder die zweite Steuereinheit zum Speichern von Informationen bzgl. der Aerosol-Verabreichung auf einem Datenspeicher des Geräts eingerichtet, wie etwa Informationen zur Dauer, Häufigkeit und/oder Menge der Aerosol-Verabreichung, und/oder zum Speichern von Informationen zum Füllstand des Behälters und/oder zum Ladestand einer Batterie. Optional kann die erste Steuereinheit und/oder die zweite Steuereinheit zur Kommunikation über ein Netzwerk, bspw. über WLAN, und/oder zur (direkten oder indirekten) Kommunikation mit einem externen Datenverarbeitungsgerät (wie etwa einem Smartphone) über einen oder mehrere bekannte Kommunikationsstands, wie etwa Bluetooth oder WLAN, eingerichtet sein. Optional ist das Gerät zur Kommunikation mit einem externen Datenverarbeitungsgerät, wie etwa einem Mobilgerät (bspw. einem Smartphone), bspw. über Bluetooth oder WLAN, eingerichtet. Zum Beispiel kann eine externe Applikation auf einem externen Datenverarbeitungsgerät Informationen abfragen, die auf einem Datenspeicher des offenbarungsgemäße Geräts abgelegt sind. Insbesondere kann das offenbarungsgemäße Gerät zur Interaktion mit einer externen Applikation, die bspw. auf einem Smartphone installiert werden kann, eingerichtet sein. Die Kommunikation kann bidirektional sein. Das Gerät kann zum Beispiel eine Push-Nachricht an eine externe Applikation auf einem Smartphone veranlassen oder selbst übermitteln, bspw. wenn eine Aerosol-Verabreichung ansteht. Ein Benutzer kann zum Beispiel über eine externe Applikation auf einem Smartphone Informationen abfragen, bspw. wann oder wie oft er bereits ein Aerosol an den Nasenraum verabreicht hat, oder wie der aktuelle Füllstand des Behälters ist. Das Smartphone kann die entsprechende Information dann mittels drahtloser Kommunikation von dem Gerät anfordern, und das Gerät kann die Information mittels drahtloser Kommunikation an das Smartphone übermitteln, bspw. wenn sie auf einem Datenspeicher des Geräts abgespeichert ist. Optional kann das Gerät alternativ oder zusätzlich eine Schnittstelle zur kabelgebundenen Kommunikation mit einem externen Datenverarbeitungsgerät aufweisen, bspw. eine USB-Schnittstelle, insbesondere eine USB-Buchse. Die Schnittstelle kann optional auch zum Laden eines Energiespeichers, insbesondere einer Batterie, des Geräts genutzt werden, zum Beispiel wenn die Schnittstelle eine USB-Schnittstelle ist.

Wie in den Figuren 1-3 dargestellt ist, kann die Aktivierungsvorrichtung 120 ein erstes Verbindungsteil 131, bspw. ein erstes magnetisches Element, zum Anbringen der Aktivierungsvorrichtung 120 an der Aerosol-Erzeugungsvorrichtung 110 aufweisen. Die Aerosol-Erzeugungsvorrichtung 110 kann ein zweites Verbindungsteil 132, bspw. ein zweites magnetisches Element, zum Koppeln an das erste Verbindungsteil 131 aufweisen. Beispielsweise sind die Aktivierungsvorrichtung 120 und die Aerosol-Erzeugungsvorrichtung 110 in dem in FIG. 3 dargestellten gekoppelten Zustand aufgrund einer magnetischen Anziehungskraft zwischen dem ersten Verbindungsteil 131 und dem zweiten Verbindungsteil 132 aneinander gehalten.

Die Aktivierungsvorrichtung 120 kann zumindest einen Haltebereich 135 zum Halten der Aktivierungsvorrichtung mit der Hand aufweisen. Die in FIG. 1 abgebildete Aktivierungsvorrichtung 120 weist zwei entgegengesetzt angeordnete Haltevertiefungen zum Eingriff jeweils eines oder mehrerer Finger auf.

Die Aerosol-Erzeugungsvorrichtung 110 weist einen Zerstäuber 111 zum Erzeugen des Aerosols 10 auf, das über eine Ausgabeöffnung 113 an den Nasenraum einer Person abgebbar ist. Die Aerosol-Erzeugungsvorrichtung 110 weist ferner optional die zweite Steuereinheit 112 zum Auslösen der Aerosol-Erzeugung durch den Zerstäuber 111 als Reaktion auf den Empfang des Aktivierungssignals auf.

FIG. 4 zeigt das offenbarungsgemäße Gerät 100 bei Verwendung in einem separierten Zustand, in dem die Aktivierungsvorrichtung 120 und die Aerosol-Erzeugungsvorrichtung 110 nicht aneinander angebracht sind. Eine Person atmet gerade in die Einblasöffnung 121 aus, bis mittels eines Drucksensors 127 ein intra-oraler Überdruck festgestellt wird, der einen Sender 125 der ersten Steuereinheit zum Versenden des Aktivierungssignals 150 veranlasst.

Das Aktivierungssignal 150 wird von der Aerosol-Erzeugungsvorrichtung 110 empfangen, woraufhin die Aerosol-Erzeugung durch den Zerstäuber 111 ausgelöst wird. Das Aerosol 10 wird über ein die Ausgabeöffnung 113 ausbildendes Nasen-Eingriffsstück 140 in die Nase der Person abgegeben. Wenn kein Aktivierungssignal mehr von der Aerosol-Erzeugungsvorrichtung 110 erfasst wird, kann die Aerosol-Erzeugung oder die Aerosol-Abgabe gestoppt werden.

Der Zerstäuber 111 kann einen der hierin beschriebenen Mechanismen zur Aerosol-Erzeugung verwenden. Vorzugsweise umfasst der Zerstäuber 111 einen Verdampfer, der das Aerosol mittels Verdampfung einer Substanz an einem Coil erzeugt. Der Verdampfer kann nach Art des elektronischen Verdampfers eine E-Zigarette aufgebaut sein. Alternativ umfasst der Zerstäuber 111 einen Ultraschallerzeuger zur Verneblung der Substanz durch Vibration, insbesondere nach Art des Ultraschall-Verneblers einer E-Zigarette.

Der Behälter 117 für die Substanz kann nach Art eines Tanks für das Liquid einer E-Zigarette aufgebaut sein. Die Substanz kann dem Zerstäuber analog zu einer E-Zigarette zugeführt werden, nämlich bspw. passiv (zum Beispiel mittels Kapillareffekt, etwa über einen Docht) oder aktiv (zum Beispiel mittels einer Fördereinrichtung, etwa einer Mikropumpe). Der Aufbau und die Funktionsweise von Zerstäubern ("Atomizer") von E-Zigaretten sind dem Fachmann bekannt, und es wird in diesem Zusammenhang auf die entsprechende Literatur verwiesen. Es sind jedoch auch andere Arten von Zerstäubern vorstellbar, wie etwa die hierin beschriebenen Spray-Zerstäuber, bspw. mittels eines unter Druck stehenden Gases und/oder mittels einer Düse.

In FIG. 4 ist ferner dargestellt, dass die Aktivierungsvorrichtung 120 optional ein Batterie-Aufnahmefach zur Aufnahme eines ersten Energiespeichers 126 in Form einer Batterie zum Bereitstellen von Energie für den Drucksensor und/oder die erste Steuereinheit aufweisen kann. Die Aerosol-Erzeugungsvorrichtung 110 kann optional ein Batterie-Aufnahmefach zur Aufnahme eines zweiten Energiespeichers 116 in Form einer Batterie zum Bereitstellen von Energie für den Zerstäuber und/oder die zweite Steuereinheit aufweisen. Entsprechend kann ein einfach handhabbares und mobiles Gerät mit verbesserten Funktionalitäten bereitgestellt werden.

Das Gerät 100 kann klein, kompakt und einfach handhabbar sein. Beispielsweise ist die maximale Abmessung des Geräts 100 im gekoppelten Zustand (also wenn die Aktivierungsvorrichtung 120 und die Aerosol-Erzeugungsvorrichtung 110 aneinander angebracht sind, siehe FIG. 3) 20 cm oder weniger, vorzugsweise 15 cm oder weniger, insbesondere 12 cm oder weniger. Die maximale Abmessung kann einer "Höhe H" des Geräts entsprechen und wird entlang der Längsachse L des Geräts gemessen, entlang der die Aktivierungsvorrichtung 120 und die Aerosol-Erzeugungsvorrichtung 110 im gekoppelten Zustand nebeneinander angeordnet sind. Die Höhe der Aktivierungsvorrichtung 120 kann 8 cm oder weniger, vorzugsweise 6 cm oder weniger, insbesondere 4 cm oder weniger betragen. Die Höhe der Aerosol-Erzeugungsvorrichtung 110 kann 12 cm oder weniger, vorzugsweise 10 cm oder weniger, insbesondere 7 cm oder weniger betragen.

Eine Breite bzw. eine Tiefe der Aktivierungsvorrichtung 110 können im Wesentlichen einer Breite bzw. einer Tiefe der Aerosol-Erzeugungsvorrichtung 120 entsprechen, so dass im gekoppelten Zustand ein kompaktes und einfach handhabbares Gerät bereitgestellt ist, wenn die Aktivierungsvorrichtung 110 in Richtung der Längsachse L auf die Aerosol-Erzeugungsvorrichtung 120 aufgesetzt und daran gekoppelt ist (siehe FIG. 3). Beispielsweise ist die Breite B des Geräts 5 cm oder weniger, insbesondere 4 cm oder weniger, und/oder die Tiefe des Geräts (gemessen senkrecht zur Papierebene der Zeichnungen) ist 3 cm oder weniger, insbesondere 2 cm oder weniger. Eine Querschnittsform des Geräts (senkrecht zur Längsachse L) kann im Wesentlichen rechteckig mit gerundeten Ecken oder oval sein, bspw. nach Art einer E-Zigarette der vierten Generation in "Boxoptik".

Die Aktivierungsvorrichtung 120 kann einen in Richtung der Aerosol-Erzeugungsvorrichtung 110 weisenden Ausschnitt bzw. eine schräg verlaufende Aussparung haben, die in der gekoppelten Stellung Raum für einen Aerosol-Ausgabekanal der Aerosol-Erzeugungsvorrichtung 110 bietet, der in Richtung der Aktivierungsvorrichtung 120 von dem Zerstäuber vorstehen kann. Die Aussparung kann im Wesentlichen komplementär zu dem Aerosol-Ausgabekanal geformt sein, so dass sich Aktivierungsvorrichtung 120 und Aerosol-Erzeugungsvorrichtung 110 im gekoppelten Zustand im Wesentlichen zu einer (abgerundeten oder eckigen) "Box" ergänzen (siehe FIG. 1-3).

Die im Querschnitt flache Form der Aktivierungsvorrichtung 110 führt bei einer Verdrehung des Mundstücks 128 relativ zu dem Grundkörper 129 um bspw. 90° zum Aktivieren der Aktivierungsvorrichtung 110 dazu, dass der durch das Mundstück ausgebildete rohrförmige Abschnitt nach unten hin geöffnet wird. Die von der Person in das Mundstück ausgeatmete Luft kann durch den Blaswiderstand 123 strömen und am unteren Ende des Mundstücks an dem Grundkörper vorbei wieder aus der Aktivierungsvorrichtung 110 hinaus in die Umgebungsluft abgegeben werden.

Das erfindungsgemäße Gerät bietet eine zuverlässige Auslösung einer Aerosol-Abgabe an den Nasenraum bei geschlossenem Gaumensegel, was für medizinische Anwendungen besonders vorteilhaft ist, und ist gleichzeitig äußerst kompakt, mobil und einfach in der Handhabung.

## Patentansprüche

1. Gerät (100) zur Verabreichung eines Aerosols (10) an den Nasenraum,
umfassend:
eine Aerosol-Erzeugungsvorrichtung (110) mit einem Zerstäuber (111) zum Erzeugen des Aerosols; und
eine Aktivierungsvorrichtung (120), umfassend:
eine Einblasöffnung (121);
eine erste Steuereinheit (122), die eingerichtet ist zur Übermittlung eines Aktivierungssignals an die Aerosol-Erzeugungsvorrichtung (110) als Reaktion auf ein Ausatmen einer Person in die Einblasöffnung (121); und
einen Blaswiderstand (123), der dazu eingerichtet ist, dem Ausatmen der Person einen Widerstand entgegenzusetzen,
**dadurch gekennzeichnet, dass**
die erste Steuereinheit (122) zum drahtlosen Aktivieren der Aerosol-Erzeugungsvorrichtung eingerichtet ist,
wobei die erste Steuereinheit (122) einen integrierten Schaltkreis (124) und/oder einen Sender (125) zum Senden des Aktivierungssignals an die Aerosol-Erzeugungsvorrichtung (110) aufweist,
wobei die Aerosol-Erzeugungsvorrichtung eine zweite Steuereinheit (112) aufweist, die eingerichtet ist zum Auslösen einer Aerosol-Erzeugung durch den Zerstäuber bei Empfang des Aktivierungssignals von der Aktivierungsvorrichtung,
wobei die Aerosol-Erzeugungseinrichtung einen Sensor aufweist, der dazu eingerichtet ist, eine Position und/oder Lage der Aerosol-Erzeugungseinrichtung in Bezug zu der Nase bzw. der Nasenöffnung des Verwenders zu bestimmen,
wobei der Sensor ausgewählt ist aus der Gruppe bestehend aus Abstandssensor, Entfernungssensor, Lagesensor, und Kombinationen davon,
wobei die Aktivierungsvorrichtung (120) und die Aerosol-Erzeugungsvorrichtung (110) bei Verwendung körperlich getrennt voneinander anordenbar sind, und
wobei die Aktivierungsvorrichtung (120) einen Drucksensor (127) aufweist, der eingerichtet ist zum Erfassen eines Drucks oder einer Druckänderung in der Aktivierungsvorrichtung.

2. Gerät (100) nach Anspruch 1, wobei die erste Steuereinheit (122) zum drahtlosen Aktivieren der Aerosol-Erzeugungsvorrichtung über ein Funk- oder IR-Signal eingerichtet ist.

3. Gerät nach einem der vorhergehenden Ansprüche, wobei die Aktivierungsvorrichtung ein die Einblasöffnung ausbildendes Mundstück (128) aufweist, durch das die Person in die Einblasöffnung ausatmen kann.

4. Gerät (100) nach Anspruch 3, wobei das Mundstück (128) um eine Längsachse (L) drehbar an einem Grundkörper (129) der Aktivierungsvorrichtung angeordnet ist, wobei eine Verdrehung des Mundstücks (128) um einen vorgegebenen Winkel die Aktivierungsvorrichtung anschaltet.

5. Gerät (100) nach einem der vorhergehenden Ansprüche, wobei die Aerosol-Erzeugungsvorrichtung ferner aufweist:
einen zur Aufnahme einer Substanz eingerichteten Behälter (117), wobei der Zerstäuber (111) zur Überführung der Substanz in das Aerosol (10) eingerichtet ist, und
20 eine Ausgabeöffnung (113), durch die das Aerosol an den Nasenraum der
Person verabreichbar ist,
wobei eine die Ausgabeöffnung (113), den Zerstäuber (111) und/oder den Behälter (117) aufweisende erste Untereinheit der Aerosol-Erzeugungsvorrichtung (110) lösbar mit einer eine zweite Steuereinheit (112) aufweisenden zweiten Untereinheit der Aerosol-Erzeugungsvorrichtung (110) verbunden ist.

6. Gerät (100) nach einem der vorhergehenden Ansprüche, wobei die Aerosol-Erzeugungsvorrichtung ein die Ausgabenöffnung ausbildendes Nasen-Eingriffsstück (140) aufweist, wobei das Nasen-Eingriffsstück (140) um eine Drehachse drehbar an der Aerosol-Erzeugungsvorrichtung (110) angeordnet ist.

7. Gerät (100) nach einem der vorhergehenden Ansprüche, wobei der Zerstäuber (111) eingerichtet ist zum Überführen einer Substanz in das Aerosol (10) durch zumindest einen der folgenden Mechanismen:
- Heizen der Substanz über ein Heizelement;
- Versetzen der Substanz in Schwingungen über ein Ultraschallelement;
- Leiten eines komprimierten Gases durch die Substanz;
- mittels einer Schwingmembran;
- Sprühen der Substanz aus einer Düse, insbesondere unter Verwendung eines Treibmittels unter Druck; und
- mittels eines Inhalationsgeräts.

8. Gerät (100) nach einem der vorhergehenden Ansprüche, wobei die Aerosol-Erzeugungsvorrichtung (110) zumindest ein Heizelement zum Heizen des Aerosols auf eine vorgegebene Temperatur aufweist, um die Abgabe des Aerosols an den Nasenraum unter einer vorgegebenen Abgabetemperatur zu ermöglichen.

9. Gerät (100) nach einem der vorhergehenden Ansprüche, wobei die Aktivierungsvorrichtung (120) ein erstes Verbindungsteil (131) zum lösbaren Anbringen an der Aerosol-Erzeugungsvorrichtung (110) aufweist, wobei die Aerosol-Erzeugungsvorrichtung (110) ein zweites Verbindungsteil (132) zum lösbaren Verbinden mit der Aktivierungsvorrichtung (120) aufweist, wobei das erste Verbindungsteil (131) mindestens ein erstes magnetisches Element aufweist, wobei das zweite Verbindungsteil (132) mindestens ein zweites magnetisches Element aufweist, wobei das erste magnetische Element mit dem zweiten magnetischen Element koppeln kann.

10. Gerät nach einem der vorhergehenden Ansprüche, wobei der Sensor der Aerosol-Erzeugungseinrichtung einen Lagesensor, insbesondere ein Magnetometer oder Gyroskop, zum Bestimmen einer Lage bzw. Orientierung der Aerosol-Erzeugungseinrichtung umfasst.

11. Gerät nach einem der vorhergehenden Ansprüche, wobei die Aerosol-Erzeugungsvorrichtung einen Luftstrombeschleuniger aufweist, zum Bereitstellen einer vorgegebenen Aerosol-Flussrate und/oder Flussgeschwindigkeit.

12. Gerät nach einem der vorhergehenden Ansprüche, wobei die Aktivierungsvorrichtung eine Anzeige aufweist, die eingerichtet ist zum Erzeugen eines Anzeigesignals bei Erreichen eines vorgegebenen Drucks beim Ausatmen der Person in die Einblasöffnung, insbesondere zum Erzeugen eines visuellen, akustischen und/oder taktilen Signals.

13. Gerät nach einem der vorhergehenden Ansprüche, wobei die Aktivierungsvorrichtung zumindest einen Haltebereich (135) zum Ergreifen und Halten der Aktivierungsvorrichtung mit einer Hand oder einem Teil davon aufweist.

14. Gerät nach Anspruch 13, wobei der zumindest eine Haltebereich (135) zumindest eine an den Eingriff eines Fingers beim Halten der Aktivierungsvorrichtung angepasste Vertiefung aufweist.

15. Gerät nach einem der vorhergehenden Ansprüche, wobei die Aerosol-Erzeugungsvorrichtung eine Anzeige aufweist, die eingerichtet ist zum Erzeugen eines Anzeigesignals, das einen Operationszustand der Aerosol-Erzeugungsvorrichtung anzeigt.

## Claims

1. An apparatus (100) for administering an aerosol (10) to the nasal cavity,
comprising:
an aerosol generating device (110) having an atomizer (111) for generating the aerosol; and
an activating device (120) comprising:
a blow-in opening (121);
a first control unit (122) adapted to transmit an activation signal to the aerosol generating device (110) in response to an exhalation of a person into the blow-in opening (121); and
a blowing resistor (123) configured to resist the person's exhalation a resistance,
**characterized in that**
the first control unit (122) is adapted to wirelessly activate the aerosol generating device,
wherein the first control unit (122) comprises an integrated circuit (124) and/or a transmitter (125) for transmitting the activation signal to the aerosol generating device (110),
wherein the aerosol generating device comprises a second control unit (112) adapted to trigger aerosol generation by the atomizer upon receipt of the activation signal from the activating device,
wherein the aerosol generating device comprises a sensor adapted to determine a position and/or location of the aerosol generating device in relation to the nose or nasal opening of the user,
wherein the sensor is selected from the group consisting of a distance sensor, a range sensor, and a position sensor, and combinations thereof,
wherein the activating device and the aerosol generating device are physically separated from each other during use, and
wherein the activating device comprises a pressure sensor (127) adapted to detect a pressure or a pressure change in the activating device.

2. The apparatus (100) according to claim 1, wherein the first control unit (122) is adapted to wirelessly activate the aerosol generating device via a radio or IR signal.

3. The apparatus according to any one of the preceding claims, wherein the activating device has a mouthpiece (128) forming the blow-in opening, through which the person can exhale into the blow-in opening.

4. The apparatus (100) according to claim 3, wherein the mouthpiece (128) is arranged on a base body (129) of the activating device so as to be rotatable about a longitudinal axis (L), wherein a rotation of the mouthpiece (128) by a predetermined angle switches on the activating device.

5. The apparatus (100) according to any one of the preceding claims, wherein the aerosol generating device further comprises:
a container (117) adapted to contain a substance, wherein the atomizer (111) is adapted to transfer the substance into the aerosol (10); and
a dispensing opening (113) through which the aerosol can be administered to the nasal cavity of the person,
wherein a first subunit of the aerosol generating device comprising the dispensing opening (113), the atomizer (111) and/or the container (117) is detachably connected to a second subunit of the aerosol generating device (110) comprising a second control unit (112).

6. The apparatus (100) according to any one of the preceding claims, wherein the aerosol generating device comprises a nose engagement piece (140) forming the dispensing opening, wherein the nose engaging piece (140) is arranged on the aerosol generating device (110) so as to be rotatable about an axis of rotation.

7. The apparatus according to any one of the preceding claims, wherein the atomizer (111) is adapted to transfer a substance into the aerosol (10) by at least one of the following mechanisms:
- heating of the substance via a heating element;
- set the substance into vibration via an ultrasonic element;
- flowing of a compressed gas through the substance;
- by means of a vibrating diaphragm;
- spraying the substance from a nozzle, in particular using a propellant under pressure; and
- by means of an inhalation device.

8. The apparatus (100) according to any one of the preceding claims, wherein the aerosol generating device comprises at least one heating element for heating the aerosol to a predetermined temperature for allowing delivery the aerosol to the nasal cavity at a predetermined delivery temperature.

9. The apparatus (100) according to any one of the preceding claims, wherein the activation device (120) has a first connecting part (131) for detachably attaching to the aerosol generating device (110), wherein the aerosol generating device (110) comprises a second connecting part (132) for detachably connecting to the activation device (120), wherein the first connecting part (131) comprises at least one first magnetic element, wherein the second connecting part (132) comprises at least one second magnetic element, wherein the first magnetic element is adapted to couple to the second magnetic element.

10. The apparatus according to any one of the preceding claims, wherein the sensor of the aerosol generating device comprises a position sensor, in particular a magnetometer or gyroscope, for determining a position and/or orientation of the aerosol generating device.

11. The apparatus according to any one of the preceding claims, wherein the aerosol generating device comprises an airflow accelerator for providing a predetermined aerosol flow rate and/or flow velocity.

12. The apparatus according to any one of the preceding claims, wherein the activating device has a display that is set up to generate a display signal when a predetermined pressure is reached when the person exhales into the blow-in opening, in particular to generate a visual, acoustic, and/or tactile signal.

13. The apparatus according to any one of the preceding claims, wherein the activating device comprises at least one holding region (135) for gripping and holding the activating device with a hand or a part thereof.

14. The apparatus according to claim 13, wherein the at least one holding region (135) has at least one recess adapted to the engagement of a finger when holding the activation device.

15. The apparatus according to any one of the preceding claims, wherein the aerosol generating device comprises an indicator adapted to generate an indicator signal indicating an operational state of the aerosol generating device.

## Revendications

1. Dispositif (100) pour administrer un aérosol (10) dans la cavité nasale, comprenant :
un dispositif de génération d'aérosol (110) avec un atomiseur (111) pour générer l'aérosol ; et
un dispositif d'activation (120) comprenant :
une ouverture d'insufflation (121) ;
une première unité de commande (122) agencée pour transmettre un signal d'activation au dispositif de génération d'aérosol (110) en réponse à l'expiration d'une personne dans l'ouverture d'insufflation (121); et
une résistance au souffle (123) agencée pour opposer une résistance à l'expiration de la personne,
la première unité de commande (122) étant agencée pour activer sans fil le dispositif de génération d'aérosol,
la première unité de commande (122) comprenant un circuit intégré (124) et/ou un émetteur (125) pour envoyer le signal d'activation au dispositif de génération d'aérosol (110),
le dispositif de génération d'aérosol comprenant une deuxième unité de commande (112) conçue pour déclencher une génération d'aérosol par l'atomiseur à la réception du signal d'activation provenant du dispositif d'activation,
le dispositif de génération d'aérosol comprenant un capteur qui est conçu pour déterminer une position et/ou un emplacement du dispositif de génération d'aérosol par rapport au nez ou à l'ouverture nasale de l'utilisateur,
le capteur étant choisi parmi le groupe comprenant un capteur de distance, un capteur d'éloignement, un capteur de position, et des combinaisons de ceux-ci,
le dispositif d'activation (120) et le dispositif de génération d'aérosol (110) pouvant être disposés physiquement séparément l'un de l'autre lors de l'utilisation, et
le dispositif d'activation (120) comportant un capteur de pression (127) qui est conçu pour détecter une pression ou une variation de pression dans le dispositif d'activation.

2. Dispositif (100) selon la revendication 1, dans lequel la première unité de commande (122) est conçue pour activer sans fil le dispositif de génération d'aérosol via un signal radio ou infrarouge.

3. Dispositif selon l'une des revendications précédentes, dans lequel le dispositif d'activation comprend un embout buccal (128) formant l'ouverture d'insufflation, à travers lequel la personne peut expirer dans l'ouverture d'insufflation.

4. Dispositif (100) selon la revendication 3, dans lequel l'embout buccal (128) est disposé de manière rotative autour d'un axe longitudinal (L) sur un corps de base (129) du dispositif d'activation, une rotation de l'embout buccal (128) d'un angle prédéfini activant le dispositif d'activation.

5. Dispositif (100) selon l'une des revendications précédentes, dans lequel le dispositif de génération d'aérosol
comprend en outre :
un récipient (117) conçu pour recevoir une substance, l'atomiseur (111) étant conçu pour transférer la substance dans l'aérosol (10) ; et
une ouverture de sortie (113) à travers laquelle l'aérosol peut être administré à la cavité nasale de la personne,
dans lequel une première sous-unité du dispositif de génération d'aérosol (110) comprenant l'ouverture de sortie (113), l'atomiseur (111) et/ou le récipient (117), est reliée de manière amovible à une deuxième sous-unité du dispositif de production d'aérosol (110) comportant une deuxième unité de commande (112).

6. Dispositif (100) selon l'une des revendications précédentes, dans lequel le dispositif de génération d'aérosol comprend une pièce d'engagement nasale (140) formant l'ouverture de sortie, la pièce d'engagement nasale (140) étant disposée de manière rotative autour d'un axe de rotation sur le dispositif de génération d'aérosol (110).

7. Dispositif (100) selon l'une des revendications précédentes, dans lequel l'atomiseur (111) est conçu pour transférer une substance dans l'aérosol (10) par au moins l'un des mécanismes suivants :
- chauffage de la substance via un élément chauffant;
- mise en vibration de la substance via un élément ultrasonique;
- conduite d'un gaz comprimé à travers la substance;
- au moyen d'une membrane vibrante;
- pulvérisation de la substance à partir d'une buse, en particulier à l'aide d'un propulseur sous pression ; et
- au moyen d'un dispositif d'inhalation.

8. Dispositif (100) selon l'une des revendications précédentes, dans lequel le dispositif de génération d'aérosol (110) comprend au moins un élément chauffant pour chauffer l'aérosol à une température prédéterminée afin de permettre la délivrance de l'aérosol dans la cavité nasale à une température de délivrance prédéterminée.

9. Dispositif (100) selon l'une des revendications précédentes, dans lequel le dispositif d'activation (120) comprend un premier élément de connexion (131) destiné à être fixé de manière amovible au dispositif de génération d'aérosol (110), le dispositif de génération d'aérosol (110) comprenant un deuxième élément de connexion (132) destiné à être relié de manière amovible au dispositif d'activation (120), le premier élément de connexion (131) comportant au moins un premier élément magnétique, le deuxième élément de connexion (132) comportant au moins un deuxième élément magnétique, le premier élément magnétique pouvant s'accoupler avec le deuxième élément magnétique.

10. Dispositif selon l'une des revendications précédentes, dans lequel le capteur du dispositif de génération d'aérosol comprend un capteur de position, en particulier un magnétomètre ou un gyroscope, pour déterminer une position ou une orientation du dispositif de génération d'aérosol.

11. Dispositif selon l'une des revendications précédentes, dans lequel le dispositif de génération d'aérosol comprend un accélérateur de flux d'air pour fournir un débit et/ou une vitesse d'écoulement d'aérosol prédéterminés.

12. Dispositif selon l'une des revendications précédentes, dans lequel le dispositif d'activation comprend un indicateur qui est conçu pour générer un signal d'indication lorsqu'une pression prédéterminée est atteinte lorsque la personne expire dans l'ouverture d'insufflation, en particulier pour générer un signal visuel, acoustique et/ou tactile.

13. Dispositif selon l'une des revendications précédentes, dans lequel le dispositif d'activation comporte au moins une zone de préhension (135) pour saisir et maintenir le dispositif d'activation d'une main ou d'une partie de celle-ci.

14. Dispositif selon la revendication 13, dans lequel la au moins une zone de préhension (135) comporte au moins un renfoncement adapté à l'engagement d'un doigt lors de la préhension du dispositif d'activation.

15. Dispositif selon l'une des revendications précédentes, dans lequel le dispositif de génération d'aérosol comprend un indicateur qui est agencé pour générer un signal d'indication indiquant un état de fonctionnement du dispositif de génération d'aérosol.
